# EUROPEAN PATENT SPECIFICATION

(11) **EP 2 934 485 B1**
(45) Date of publication and mention of the grant of the patent: **29.05.2019**
(21) Application number: 13814126.2
(22) Date of filing: 19.12.2013
(51) Int. Cl.: A61K 9/14, A61K 31/00, A61K 9/20, A61K 31/135

(54) **TABLET COMPOSITION COMPRISING CINACALCET HYDROCHLORIDE**
TABLETTENZUSAMMENSETZUNG MIT CINACALCET-HYDROCHLORID
COMPOSITION DE COMPRIMÉ COMPRENANT DU CHLORHYDRATE DE CINACALCET

(30) Priority: 21.12.2012 WO PCT/EP2012/076732
(43) Date of publication of application: 28.10.2015
(73) Proprietor: Synthon B.V., 6545 CM Nijmegen (NL)
(72) Inventor: MURPANI, Deepak, 6545 CM Nijmegen (NL); VIVANCOS MARTINEZ, Marta, 08830 Sant Boi de Llobregat (ES)
(74) Representative: Tejedor Vinent, Henar
(86) International application number: PCT/EP2013/077523
(87) International publication number: WO 2014/096277

(56) References cited:
- WO-A1-2005/034928
- WO-A1-2013/107503
- WO-A2-2008/068625
- WO-A2-2010/034497
- WO-A2-2011/146583

## Description

### BACKGROUND OF THE PRESENT INVENTION

The present invention relates to a pharmaceutical composition, particularly a tablet composition comprising a high drug load of cinacalcet hydrochloride, having improved bioavailability.

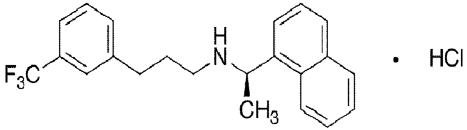

Cinacalcet, having the chemical structure shown above, is a calcimimetic agent, and as such it increases the sensitivity to extracellular calcium of the calcium-sensing receptors of the parathyroid gland, leading to reduced levels of parathyroid hormone, serum calcium, and phosphate. It is indicated for the treatment of secondary hyperparathyroidism in patients with chronic kidney disease on dialysis.

Cinacalcet free base and pharmaceutically acceptable salts thereof are disclosed in European Patent application EP1203761.

A cinacalcet hydrochloride-containing pharmaceutical product is approved in many countries of the world under the brand name Mimpara® (Amgen) in the EU and Sensipar® (Amgen) in the US. Generally, the marketed cinacalcet hydrochloride tablets comprise 30, 60 or 90 mg of cinacalcet hydrochloride.

Cinacalcet hydrochloride is poorly soluble in water and has a pH dependent solubility. At basic pH it is practically insoluble (<0.001 mg/ml). The solubility is about 1.6 mg/ml at a pH range of from about 3-5, however at pH 1 the solubility decreases to approx. 0.1 mg/ml. In the state of the art, various proposals have been made on how to formulate this active pharmaceutical ingredient.

The marketed formulation of cinacalcet hydrochloride is described in WO2005034928. The tablet composition disclosed therein contains cinacalcet hydrochloride with a particle size distribution D₅₀ less than or equal to 50 µm (see paragraph [021]) in an amount of approx. 18% by weight based on the total weight of the tablet composition (see paragraphs [019] and [057]).

The tablet of WO2005034928 contains a high amount (i.e. 70% by weight) of diluent among other pharmaceutical excipients, having a tablet weight of up to 540 mg for the cinacalcet 90 mg tablet strength. The incorporation of a relatively high amount of excipients (e.g., diluents and disintegrants) into the formulation of a solid oral dosage form can improve the dissolution rate of active pharmaceutical ingredients, especially those that are relatively hydrophobic and poorly soluble in water like cinacalcet. Increasing the amount of excipients in the composition, however, entails a number of disadvantages. Notably, tablet size will increase significantly causing patient compliance problems. In addition, content uniformity can be problematic as well as stability problems can occur related to interaction of the active pharmaceutical ingredient with one or more of the excipients.

The objective of the present invention was therefore to overcome the above-mentioned disadvantages.

### DETAILED DESCRIPTION OF THE PRESENT INVENTION

The present invention relates to a pharmaceutical composition containing a high amount of cinacalcet hydrochloride having a particle size distribution D₉₀ equal to or less than 30 µm, preferably equal to or less than 25 µm, more preferably equal to or less than 10 µm, exhibiting good drug release and bioavailability profile. The preferred tablet composition has a high drug load, a reduced size and is bioequivalent as compared to the commercially available tablet.

In one embodiment, the present invention relates to a tablet composition comprising a therapeutically effective dose of cinacalcet hydrochloride having a particle size distribution D₉₀ equal to or less than 30 µm, preferably equal to or less than 25 µm, more preferably equal to or less than 10 µm, in an amount of from 40% to 60%, preferably from 45% to 55% by weight based on the total weight of the composition, and one or more pharmaceutically acceptable excipients.

The D₉₀ value of the particle size distribution is defined as the particle diameter at which 90% by volume of the particles have a smaller diameter than the diameter which corresponds to the D₉₀ value measured by laser diffractometry. Specifically, a Malvern Instruments Mastersizer was used to determine the particle size distribution.

The one or more pharmaceutically acceptable excipients to be used in accordance with the present invention can be chosen from, for example, diluents, binders, disintegrants, lubricants, and glidants.

Diluents are fillers which are used to increase the bulk volume of a tablet or capsule. Generally, by combining a diluent with the active pharmaceutical ingredient, the final product is given adequate weight and size to assist in production and handling. Binders hold the excipients that are present in a tablet together. Binders ensure that tablets and granules can be formed having the desired or required mechanical strength, and they give volume to low active dose tablets. Suitable examples of diluents to be used in accordance with the present invention include starch, pregelatinized starch, microcrystalline cellulose, and calcium phosphate, lactose, sorbitol, mannitol and sucrose.

The tablet composition of the present invention preferably contains at least one hydrophilic diluents. Starch, pregelatinized starch, lactose, sorbitol, mannitol and sucrose are suitable hydrophilic diluents.

In a preferred embodiment of the present invention, the at least one hydrophilic diluent is pregelatinized starch.

The tablet composition of the invention preferably comprises:
a) from 30% to 50% of one or more diluents by weight based on the total weight of the composition;
b) at least one binder in an amount of from 1% to 5% by weight based on the total weight of the composition.

Binders which are suitable for use in accordance with the present invention include povidone, hydroxypropyl methylcellulose, dihydroxy propylcellulose, and sodium carboxyl methylcellulose. Binders are preferably used in an amount of from 1% to 5% by weight based on the total weight of the composition. A preferred binder is povidone.

The tablet composition of the present invention may also contain a disintegrant. Disintegrants are added to a tablet composition to promote the breakup of the tablet into smaller fragments in an aqueous environment, thereby increasing the available surface area and promoting a more rapid release of the active pharmaceutical ingredient. Suitable examples of disintegrants to be used in accordance with the present invention include crospovidone, sodium starch glycolate, croscarmellose sodium, and mixtures of any of the foregoing. Disintegrants preferably are used in an amount of from 1% to 10% by weight based on the total weight of the composition.

The tablet composition of the invention may also contain a lubricant. Lubricants are generally used in order to reduce sliding friction. In particular, to decrease friction at the interface between a tablet's surface and the die wall during ejection, and reduce wear on punches and dies. Suitable lubricants to be used in accordance with the present invention include magnesium stearate, calcium stearate, stearic acid, glyceryl behenate, hydrogenated vegetable oil, and glycerine fumarate. The tablet composition of the invention may also contain a glidant. Glidants enhance product flow by reducing interparticulate friction. A suitable example is colloidal silicon dioxide.

Lubricants and glidants preferably are used in a total amount of from 0.05% to 5% by weight based on the total weight of the composition.

In a preferred embodiment, the tablet composition of the present invention contains the following ingredients, based on the total weight of the composition:
a. A therapeutically effective dose of cinacalcet hydrochloride in an amount of from 45% to 55% by weight;
b. Microcrystalline cellulose or pregelatinized starch or a mixture thereof in an amount of from 30% to 50% by weight;
c. Povidone in an amount of from 1% to 5% by weight;
d. Crospovidone in an amount of from 1% to 10% by weight; and
e. From 0.05% to 5% by weight of a lubricant and a glidant.

In one embodiment of the present invention, the therapeutically effective dose of cinacalcet hydrochloride is 30 mg, 60 mg or 90 mg.

The pharmaceutically acceptable excipients to be used in accordance with the present invention, can be used only intragranularly, only extragranularly, or both.

The present invention further relates to a tablet composition as described hereinabove, prepared by a wet-granulation process, which process comprises:
1. Mixing cinacalcet hydrochloride and one or more pharmaceutically acceptable excipients to form a mixture;
2. Wet-granulating the resulting mixture;
3. Further mixing the obtained granulate with one or more further pharmaceutically acceptable excipients to form a further mixture;
4. Compressing the mixture obtained in step (3) into a tablet; and optionally
5. Coating the tablet.

The present invention still further relates to a granulate suitable for making a tablet composition as described hereinabove, prepared by a wet-granulation process, which process comprises:
1. Mixing cinacalcet hydrochloride and one or more pharmaceutically acceptable excipients to form a mixture, and
2. Wet-granulating the resulting mixture.

In a preferred embodiment, the granulate of the invention contains an hydrophilic diluent. More preferably it contains pregelatinized starch and optionally other hydrophilic diluents. Pregelatinized starch is an excipient that generally can act as a diluent but also as binder improving cohesion of the granulate. We have found that surprisingly by adding pregelatinized starch intragranularly to our formulation, the dissolution profile is improved. This improvement is significant when compared to other known diluents and binders. Pregelatinized starch creates a hydrophilic environment that facilitates tablet disintegration and improves the dissolution of Cinacalcet significantly more than any other similar hydrophilic diluents.

The granules of the present invention typically have a particle size distribution D₅₀ of from 200-300 µm.

The present invention also relates to a pharmaceutical composition comprising a granulate as described hereinabove in the form of a capsule or a tablet, preferably a tablet.

The pharmaceutical compositions described herein can be made using conventional methods and equipment well-known in the art.

The pharmaceutical (tablet) compositions of the present invention have a high load of cinacalcet hydrochoride and show an in vitro dissolution profile wherein at least 75% of cinacalcet is released at thirty minutes when the composition is subjected to a dissolution study in 900 ml HCl 0.05N (pH 1.3) using a USP apparatus II at 75 rpm at 37°C. Preferably, at least 85% of cinacalcet is released from the pharmaceutical composition at thirty minutes and the tablet compositions in accordance with the present invention are bioequivalent in vitro and in vivo to the commercially available cinacalcet hydrochloride tablets.

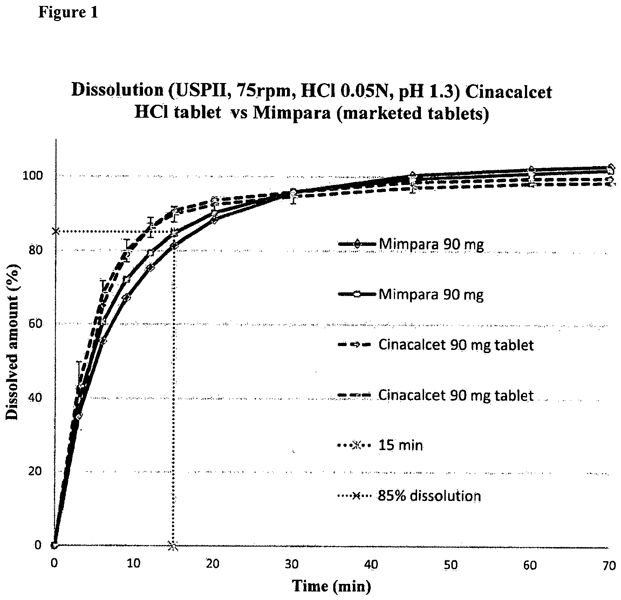

Figure 1 shows the in vitro dissolution profile of tablet compositions in accordance with the present invention as compared to commercially available tablets.

The present invention is illustrated by the following Examples.

### EXAMPLES

### Example 1

Three strengths of cinacalcet hydrochloride tablets were prepared in a conventional way as described further herein below, and have the following compositions:

| | **% (w/w)** | **30 mg** | **60 mg** | **90 mg** |
|---|---|---|---|---|
| Intragranular | | | | |
| Cinacalcet hydrochloride | 50.860 | 33.06 | 66.12 | 99.18 |
| Pregelatinized starch (Starch 1500) | 33.378 | 21.70 | 43.39 | 65.09 |
| Crospovidone (Polyplasdone XL-10) | 2.000 | 1.30 | 2.60 | 3.90 |
| Povidone (Plasdone K29/32) | 2.000 | 1.30 | 2.60 | 3.90 |
| Purified water | qs | qs | qs | qs |

| Extragranular | | | | |
|---|---|---|---|---|
| Microcrystalline cellulose (Avicel PH102) | 7.262 | 4.72 | 9.44 | 14.16 |

| | **% (w/w)** | **30 mg** | **60 mg** | **90 mg** |
|---|---|---|---|---|
| Crospovidone (Polyplasdone XL) | 3.000 | 1.95 | 3.90 | 5.85 |
| Colloidal silicon dioxide (Colloidal anhydrous silica) | 0.500 | 0.33 | 0.65 | 0.98 |
| Magnesium stearate | 1.000 | 0.65 | 1.30 | 1.95 |
| Core tablet | 100.000 | 65.00 | 130.00 | 195.00 |
| Opadry II 85F210073 | 4.000 | 2.60 | 3.35 | 7.80 |
| Coated tablet | 104.000 | 67.60 | 133.35 | 202.80 |

### Example 2

**Cinacalcet hydrochloride 90 mg tablets**

| | **% (w/w)** | **90 mg** |
|---|---|---|
| Intragranular | | |
| Cinacalcet hydrochloride | 50.09 | 99.18 |
| Pregelatinized starch (Starch 1500) | 32.87 | 65.09 |
| Crospovidone (Polyplasdone XL-10) | 2.95 | 5.8 |
| Povidone (Plasdone K29/32) | 1.97 | 3.90 |
| Purified water | qs | qs |

| Extragranular | | |
|---|---|---|
| Microcrystalline cellulose (Avicel PH102) | 7.68 | 15.21 |
| Crospovidone (Polyplasdone XL) | 2.96 | 5.85 |
| Colloidal silicon dioxide (Colloidal anhydrous silica) | 0.49 | 0.98 |
| Magnesium stearate | 0.98 | 1.95 |
| Core tablet | 100.000 | 198.00 |
| Opadry II, green | 4.000 | 7.92 |
| Coated tablet | | 205.92 |

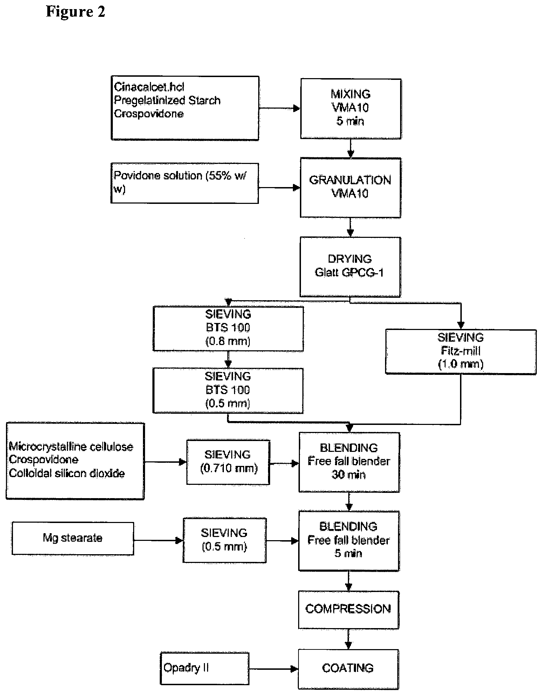

The 30, 60 and 90 mg tablets of Example 1 and 2 were made according to the process depicted in Figure 2.

Figure 1 shows the in vitro dissolution profiles of 90 mg cinacalcet hydrochloride tablets in accordance with the present invention as compared to commercially available Mimpara® 90 mg tablets.

## Claims

1. A tablet composition comprising
a) a therapeutically effective dose of cinacalcet hydrochloride having a particle size distribution D₉₀ equal to or less than 30 µm in an amount of from 45% to 55% by weight based on the total weight of the composition,
b) pregelatinized starch in an amount of from 30% to 50% by weight based on the total weight of the composition; and
c) At least one binder in an amount of from 1% to 5% by weight based on the total weight of the composition.

2. A tablet composition according to claim 1 wherein the cinacalcet hydrochloride has a D₉₀ equal to or less than 25 µm, preferably equal to or less than 10 µm.

3. A tablet composition according to any one of claims 1 to 2 wherein the composition further comprises a disintegrant in an amount of from 1% to 10% by weight based on the total weight of the composition.

4. A tablet composition according to any one of claims 1 to3, wherein the composition further comprises a lubricant and a glidant in a total amount of from 0.05% to 5% by weight based on the total weight of the composition.

5. A tablet composition according to any one of claims 1 to 4prepared by a wet-granulation process, which process comprises
1. Mixing cinacalcet hydrochloride and one or more pharmaceutically acceptable excipients to form a mixture;
2. Wet-granulating the resulting mixture;
3. Further mixing the obtained granulate with one or more further pharmaceutically acceptable excipients to form a further mixture;
4. Compressing the mixture obtained in step (iii) into a tablet; and optionally
5. Coating the tablet.

6. A tablet composition according to any one of claims 1 to 5 comprising, based on the total weight of the composition,
a) A therapeutically effective dose of cinacalcet hydrochloride in an amount of from 45% to 55% by weight;
b) pregelatinized starch in an amount of from 30% to 50% by weight;
c) Povidone in an amount of from 1% to 5% by weight;
d) Crospovidone in an amount of from 1% to 10% by weight; and
e) From 0.05% to 5% by weight of a lubricant and/or a glidant.

7. A tablet composition according to any one of claims 1 to6further comprising at least one coating material in an amount of from 1 % to 6% by weight based on the total weight of the composition.

8. A tablet composition according to any one of claims 1 to 7, wherein the therapeutically effective dose of cinacalcet hydrochloride is 30 mg, 60 mg or 90 mg.

## Patentansprüche

1. Tablettenzusammensetzung, umfassend:
a) eine therapeutisch wirksame Dosis von Cinacalcet-Hydrochlorid mit einer Partikelgrößenverteilung D₉₀ gleich oder geringer als 30 µm in einem Gewichtsanteil von 45% bis 55%, basierend auf dem Gesamtgewicht der Zusammensetzung,
b) vorgelatinierte Stärke in einem Gewichtsanteil von 30 % bis 50%, basierend auf dem Gesamtgewicht der Zusammensetzung; und
c) Zumindest ein Bindemittel in einem Gewichtsanteil von 1% bis 5 %, basierend auf dem Gesamtgewicht der Zusammensetzung.

2. Tablettenzusammensetzung nach Anspruch 1, wobei das Cinacalcet-Hydrochlorid einen D₉₀ gleich oder geringer als 25 µm, bevorzugt gleich oder geringer als 10 µm, aufweist.

3. Tablettenzusammensetzung nach einem der Ansprüche 1 bis 2, wobei die Zusammensetzung ferner ein Zerfallsmittel in einem Gewichtsanteil von 1 % bis 10 %, basierend auf dem Gesamtgewicht der Zusammensetzung, umfasst.

4. Tablettenzusammensetzung nach einem der Ansprüche 1 bis 3, wobei die Zusammensetzung ferner ein Gleitmittel und ein Antihaftmittel in einem Gesamtgewichtsanteil von 0.05% bis 5%, basierend auf dem Gesamtgewicht der Zusammensetzung, umfasst.

5. Tablettenzusammensetzung nach einem der Ansprüche 1 bis 4, hergestellt durch einen Nassgranulierungsprozess, wobei der Prozess Folgendes umfasst:
1. Mischen von Cinacalcet-Hydrochlorid und einem oder mehreren pharmazeutisch annehmbaren Trägerstoffen zum Bilden einer Mischung;
2. Nassgranulieren der resultierenden Mischung;
3. Weiteres Mischen des erhaltenen Granulats mit einem oder mehreren weiteren pharmazeutisch annehmbaren Trägerstoffen zum Bilden einer weiteren Mischung;
4. Pressen der in Schritt (iii) erhaltenen Mischung zu einer Tablette; und optional
5. Beschichten der Tablette.

6. Tablettenzusammensetzung nach einem der Ansprüche 1 bis 5, basierend auf dem Gesamtgewicht der Zusammensetzung, Folgendes umfassend:
a) Eine therapeutisch wirksame Dosis von Cinacalcet-Hydrochlorid in einem Gewichtsanteil von 45% bis 55%;
b) pregelatinierte Stärke in einem Gewichtsanteil von 30 % bis 50%;
c) Povidon in einem Gewichtsanteil von 1% bis 5%;
d) Crospovidon in einem Gewichtsanteil von 1% bis 10%; und
e) 0.05% bis 5% Gewichtsanetil eines Gleitmittels und/oder Antihaftmittels.

7. Tablettenzusammensetzung nach einem der Ansprüche 1 bis 6, ferner zumindest ein Beschichtungsmaterial in einem Gewichtsanteil von 1% bis 6%, basierend auf dem Gesamtgewicht der Zusammensetzung, umfassend.

8. Tablettenzusammensetzung nach einem der Ansprüche 1 bis 7, wobei die therapeutisch wirksame Dosis von Cinacalcet-Hydrochlorid 30 mg, 60 mg oder 90 mg beträgt.

## Revendications

1. Une composition de comprimé comprenant
a) une dose thérapeutiquement efficace de chlorhydrate de cinacalcet ayant une distribution granulométrique D₉₀ égale ou inférieure à 30 µm en une quantité de 45 % à 55 % en poids par rapport au poids total de la composition,
b) de l'amidon prégélatinisé en une quantité comprise entre 30 % et 50 % en poids par rapport au poids total de la composition ; et
c) au moins un liant en une quantité comprise entre 1 % et 5 % en poids par rapport au poids total de la composition.

2. Composition de comprimé selon la revendication 1, dans laquelle le chlorhydrate de cinacalcet a un D90 égal ou inférieur à 25 µm, de préférence égal ou inférieur à 10 µm.

3. Composition de comprimé selon l'une quelconque des revendications 1 à 2, dans laquelle la composition comprend en outre un désintégrant en une quantité comprise entre 1 % et 10 % en poids par rapport au poids total de la composition.

4. Composition de comprimé selon l'une quelconque des revendications 1 à 3, dans laquelle la composition comprend en outre un lubrifiant et un agent de glissement en une quantité totale comprise entre 0,05 % et 5 % en poids par rapport au poids total de la composition.

5. Composition de comprimé selon l'une quelconque des revendications 1 à 4 préparée par un procédé de granulation humide, lequel procédé comprend
le mélange de chlorhydrate de cinacalcet et d'un ou plusieurs excipients pharmaceutiquement acceptables pour former un mélange ;
la granulation humide du mélange obtenu ;
le mélange en plus du granulé obtenu avec un ou plusieurs autres excipients pharmaceutiquement acceptables pour former un autre mélange ;
la compression du mélange obtenu à l'étape (iii) en un comprimé ; et éventuellement
l'enrobage du comprimé.

6. Composition de comprimé selon l'une quelconque des revendications 1 à 5 comprenant, par rapport au poids total de la composition,
a) une dose thérapeutiquement efficace de chlorhydrate de cinacalcet en une quantité comprise entre 45 % et 55 % en poids ;
b) de l'amidon prégélatinisé en une quantité comprise entre 30 % et 50 % en poids ;
c) de la polyvidone en une quantité comprise entre 1 % et 5 % en poids ;
d) de la crospovidone en une quantité comprise entre 1 % et 10 % en poids ; et
e) et entre 0,05 % et 5 % en poids d'un lubrifiant et/ou d'un agent de glissement.

7. Composition de comprimé selon l'une quelconque des revendications 1 à 6, comprenant en outre au moins un matériau d'enrobage en une quantité comprise entre 1 % et 6 % en poids par rapport au poids total de la composition.

8. Composition de comprimé selon l'une quelconque des revendications 1 à 7, dans laquelle la dose thérapeutiquement efficace de chlorhydrate de cinacalcet est de 30 mg, 60 mg ou 90 mg.
